# FASCICULE DE BREVET EUROPEEN

(11) **EP 1 424 037 B1**
(45) Date de publication et mention de la délivrance du brevet: **14.06.2006**
(21) Numéro de dépôt: 03292948.1
(22) Date de dépôt: 27.11.2003
(51) Int. Cl.: A61B 5/021

(54) **Dispositif de mesure non invasive de la pression artérielle, notamment pour le suivi ambulatoire en continu de la pression artérielle**
Vorrichtung für die nicht-invasive Messung des arteriellen Blutdrucks, insbesonders für die ambulante Überwachung des arteriellen Blutdrucks
Device for non-invasive measurement of arterial pressure, especially for the continuous ambulatory tracking of arterial pressure

(30) Priorité: 29.11.2002 FR 0215069
(43) Date de publication de la demande: 02.06.2004
(73) Titulaire: ELA MEDICAL, F-92541 Montrouge (FR)
(72) Inventeur: Faisandier, Yves, 75116 Paris (FR)
(74) Mandataire: Dupuis-Latour, Dominique

(56) Documents cités:
- DE-A- 10 033 171
- DE-A- 10 061 189
- DE-A- 19 740 931

## Description

L'invention concerne un dispositif de mesure non invasive de la pression artérielle, destinée notamment au suivi ambulatoire en continu.

On connaît plusieurs techniques pour mesurer la pression artérielle.

Une première technique, qui permet une mesure en continu, consiste à introduire un cathéter intra-artériel raccordé à un capteur de pression. Cette technique, qui est par nature invasive, est utilisée principalement en réanimation, en chirurgie ou lors de certaines explorations. Son caractère invasif et le risque d'hémorragie la rendent cependant en pratique inutilisable dans un contexte ambulatoire.

Une autre technique, non invasive et couramment utilisée en ambulatoire, consiste à équiper le patient d'un brassard gonflé périodiquement. L'indication de la pression artérielle est donnée, brassard gonflé, par mesure des bruits de Korotkov (bruit émis par l'artère lorsque sa pression oscille de part et d'autre de la pression du ballon) ou par la technique de Pachon (mesure des variations de volume de l'artère par un deuxième ballon placé en aval du premier).

Cette technique fournit des mesures ponctuelles et en l'automatisant il est possible d'obtenir des valeurs de pression artérielle à certains intervalles de temps afin de reconstruire une courbe sur une durée de plusieurs heures à plusieurs jours.

Elle présente cependant un certain nombre d'inconvénients. Tout d'abord, le gonflement périodique du ballon dérange le patient et peut le réveiller la nuit. D'autre part, comme la pression n'est pas mesurée de manière continue, le risque subsiste de passer à coté d'une crise hypertensive ou hypotensive si cette crise survient entre deux mesures. Enfin, il est difficile d'imaginer un suivi ambulatoire de longue durée, pendant plusieurs jours, car la compression répétitive des tissus du membre inséré dans le ballon est insupportable pour le patient.

Une troisième technique de mesure, de nature non invasive et continue, consiste à utiliser un ballon placé autour d'un membre (en général un doigt) et gonflé en permanence à une pression asservie au volume de sang interne, volume qui est en général mesuré à l'aide d'un faisceau infrarouge traversant le doigt. On obtient ainsi une courbe continue de la pression artérielle.

Cette technique peut être miniaturisée afin d'être mise en oeuvre en ambulatoire. Pour effectuer des surveillances sur des périodes de plusieurs heures, il faut toutefois prévoir de changer fréquemment la position du ballon car les tissus supportent mal la compression permanente. Une solution proposée consiste à mettre en place deux ballons, chacun sur un doigt différent, et utiliser l'un et l'autre alternativement. La complexité de la mise en oeuvre de cette technique et son inconfort pour le patient limitent cependant son utilisation courante et sa généralisation pour un suivi ambulatoire, notamment sur une longue durée comme une journée complète ou même plusieurs jours consécutifs.

Des techniques de mesure apparentées, destinées à évaluer la pression intracardiaque, sont divulguées par le document DE-A-197 40 931. Le document met en oeuvre un capteur de pression dont les indications sont corrigées par des mesures phonocardiographiques et électrocardiographiques.

L'un des buts de l'invention est de proposer une nouvelle technique de mesure de la pression artérielle :
- qui soit par nature non invasive,
- qui délivre une mesure en continu et permanente de la pression artérielle,
- et qui ne présente pas d'inconfort pour le patient, permettant ainsi un usage en ambulatoire sans inconvénient, y compris dans les périodes de sommeil et sur des durées d'analyse pouvant atteindre plusieurs jours consécutifs.

A cet effet, l'invention propose un dispositif de mesure de la pression artérielle comprenant les caractéristiques énoncées par la revendication 1. Les sous-revendications énoncent des formes de mise en oeuvre subsidiaires avantageuses.

On va maintenant décrire un exemple de mise en oeuvre de l'invention, en référence aux dessins annexés.
La figure 1 est une vue schématique d'une réalisation à deux capteurs posés sur le thorax d'un patient.
La figure 2 illustre le signal phonocardiographique délivré par le ou les capteurs et analysé par le dispositif.

Essentiellement, l'invention consiste à mesurer la pression artérielle de manière indirecte à partir des bruits émis par le coeur

On peut reconnaître dans chaque cycle d'un coeur sain deux bruits majeurs :
- le premier bruit correspond à la fermeture des valves mitrale et accessoirement tricuspide lors du début de la contraction ventriculaire (systole),
- le second bruit correspond à la fermeture des valves aortique et accessoirement pulmonaire, à la fin de la contraction cardiaque.

Ces bruits sont recueillis par le dispositif de l'invention au moyen d'un appareillage phonocardiographique, technique qui consiste à placer sur la paroi thoracique du patient, à hauteur du coeur, un capteur permettant de transformer en signaux électriques (signaux phonocardiographiques) les signaux acoustiques engendrés principalement par la fermeture des valves cardiaques et transmis au travers du thorax.

Le capteur peut être un microphone ou, en variante, un accéléromètre présentant une bande passante suffisamment large s'étendant aux infrasons, typiquement une bande passante de l'ordre de 10 à 500 Hz.

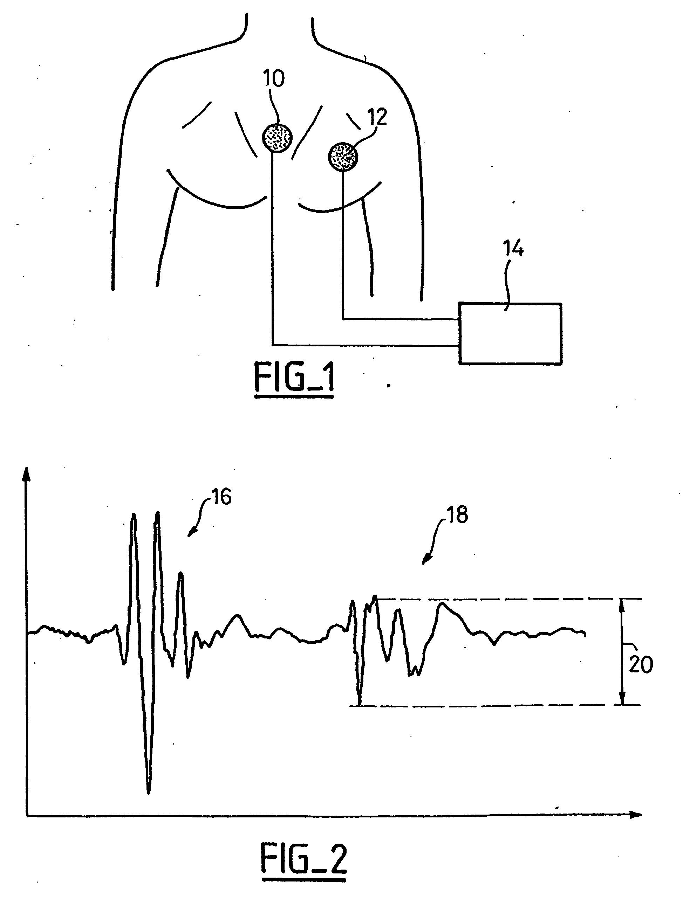

Sur la figure 1, on a illustré une configuration à deux microphones 10, 12 placés de part et d'autre du point de maximum d'amplitude et recevant donc des signaux d'amplitudes sensiblement identiques. Les signaux de ces microphones sont combinés pour donner un signal moyen stable analysé par un boîtier portatif autonome d'analyse et d'enregistrement, du même type que les dispositifs Holter utilisés pour l'enregistrement ambulatoire en continu des signaux électrocardiographiques.

Le signal obtenu est un signal présentant périodiquement, à chaque cycle cardiaque, les deux bruits majeurs indiqués plus haut, respectivement 16 et 18.

Le second bruit 18 présente une amplitude 20 qui peut varier au cours du temps.

Cette amplitude est principalement liée à l'onde de choc créée par la fermeture des valves aortiques sous l'effet de l'écart de pression entre aorte et ventricule. Lorsque le ventricule a terminé sa contraction, la pression intraventriculaire est faible, et la pression aortique correspond à la pression artérielle systolique.

Il est ainsi possible de trouver une relation entre la pression artérielle systolique et le second bruit. Par analyse de l'amplitude il est ainsi possible de délivrer une valeur, désignée ci-après "indice phonoartériel", donnant une indication relative de la valeur de la pression artérielle.

En variante ou en complément de l'analyse de l'amplitude 20 du second bruit 18, il est possible de déterminer l'indice phonoartériel à partir d'autres paramètres de ce second bruit, tels que l'énergie du signal, la variation de la dérivée du signal (notamment la valeur maximale de cette dérivée), l'aire du signal ou du pic principal de ce signal, ou une combinaison de ces paramètres.

L'analyse de l'indice phonoartériel et son enregistrement sur une longue durée permettra au praticien d'opérer un diagnostic, de reconnaître telle

ou telle pathologie, de déterminer la survenue et l'importance d'un ou plusieurs épisodes d'hypotension ou d'hypertension, etc.
Par ailleurs, le dispositif de l'invention qui, comme on l'a indiqué plus haut, donne une indication relative de la pression artérielle, peut être couplé à un autre dispositif qui, lui, réalise une mesure absolue de cette même pression artérielle, par exemple un dispositif mettant en oeuvre l'une des techniques indiquées plus haut impliquant le gonflage d'un ballon placé autour d'un membre ou d'un doigt.

Dans ce cas, le dispositif de l'invention assure un suivi en continu des variations de la pression et, en cas d'anomalie constatée (par exemple une chute brutale de la pression), il pourra déclencher la mise en oeuvre de la mesure absolue de la pression, de manière à compléter les indications qui seront fournies au praticien pour son diagnostic ; on ne risque pas ainsi passer à coté d'une crise hyper ou hypotensive si celle-ci a lieu entre deux mesures, comme dans le cas des techniques utilisant uniquement le gonflage périodique d'un ballon à intervalles prédéterminés : dans le cas présent c'est le dispositif de l'invention qui commande le gonflage du ballon, seulement si la mesure absolue de la pression est utile.

Inversement, le dispositif de l'invention peut être utilisé pour inhiber un dispositif de mesure absolue de la pression artérielle, par exemple pendant les périodes où la pression est particulièrement stable, comme dans les phases nocturnes : le gonflage périodique du brassard est inhibé pendant ces périodes et ne vient pas gêner le patient pendant son sommeil.

Le dispositif de l'invention peut être également couplé avec un dispositif, implanté ou externe, de mesure et d'analyse d'un signal électrocardiographique. En effet, du fait de l'excellente corrélation de l'indice phonoartériel avec la pression artérielle, il est par exemple possible de détecter une syncope dès son début, bien avant la bradycardie engendrée par la chute de la pression. Ainsi, la détection du début d'une syncope par le dispositif de l'invention peut par exemple permettre de déclencher immédiatement un enregistrement électrocardiographique détaillé, afin de pouvoir disposer de mesures fines du rythme cardiaque et, le cas échéant, mettre en oeuvre sans délai une thérapie appropriée (par pilotage de la prothèse intracardiaque ou délivrance d'une substance médicamenteuse).

Une application correcte de la technique de la présente invention suppose un recueil convenable du bruit cardiaque, ce recueil devant rester toujours identique quels que soient les changements positionnels du patient et les variations de son cycle respiratoire.

En particulier, dans le cas d'un recueil ambulatoire les changements positionnels du patient sont importants ; le coeur se déplace dans le thorax et les ondes acoustiques ne se propagent pas de la même manière selon que le patient est en position debout, couché, etc. De la sorte, si l'on n'effectue aucune correction les modifications corrélatives du signal risquent d'entraîner une mauvaise interprétation des variations de la pression artérielle.

Il existe plusieurs manières de remédier à cette difficulté.

Une première solution, évoquée plus haut, consiste à utiliser une pluralité de microphones (deux ou plus) placés de part et d'autre du point de maximum d'amplitude et recevant un signal d'amplitude sensiblement identique. Lors des changements positionnels l'onde de choc acoustique s'orientera plus vers l'un ou l'autre des microphones, et un calcul simple permet d'obtenir un signal moyen parfaitement stable à partir de deux ou trois signaux recueillis.

Une autre solution consiste à utiliser un élément de référence dans le signal même, par exemple l'amplitude du premier bruit 16, et normaliser l'amplitude du second bruit 18 en fonction de cet élément de référence. En cas de changement positionnel altérant les deux composantes de manière identique, on retrouvera un indice phonoartériel parfaitement stabilisé. Des mesures doivent cependant être prises pour détecter des modifications du paramètre de référence dues par exemple à une chute globale de pression par insuffisance cardiaque, ce qui conduirait à une diminution de l'amplitude de toutes les composantes, phénomène qu'il y a bien entendu lieu d'éliminer pour ne pas fausser la détermination de l'indice phonoartériel.

Une autre solution encore consiste a effectuer un apprentissage au moment de la pose des microphones, en plaçant le patient en plusieurs positions différentes et en déterminant pour chaque position un facteur correctif correspondant, qui sera appliqué ultérieurement à chaque évaluation du paramètre phonoartériel en fonction de la position du patient au moment de la mesure.

Il y a également lieu de tenir compte des mouvements respiratoires du patient, qui ne doivent pas perturber le recueil du signal phonocardiographique. Les mouvements respiratoires, en introduisant plus ou moins d'air dans les poumons qui constituent un absorbant, viennent en effet moduler de manière cyclique l'amplitude des signaux recueillis sur la paroi thoracique.

La constante de temps de cette modulation cyclique restant longue (typiquement de l'ordre de quatre secondes) par rapport aux variations du signal phonocardiographique, un simple filtrage passe-bas permet de les éliminer simplement et efficacement.

Néanmoins, si l'on veut obtenir un temps de réponse court pour le dispositif, il peut être avantageux d'appliquer un filtrage adaptatif ou bien une correction dynamique de gain. Le filtrage adaptatif peut être réalisé au moyen d'un filtre passe-bas dont la fréquence de coupure, variable, est calculée en fonction de la fréquence de la respiration, cette dernière étant reconnue à partir de la modulation cyclique d'amplitude dans la bande 10 à 20 cycles par minutes. Le filtrage dynamique, quant à lui, consiste à reconnaître les variations d'amplitude présentes au cours du cycle respiratoire et à moduler le gain en fonction de la phase au sein de ce cycle. Ces techniques sont en elles-mêmes bien connues et ne seront pas décrites plus en détail.

L'invention est notamment applicable à des enregistreurs ambulatoires externes tels que les modèles SYNEFLASH et SYNEVIEW de la société ELA Médical, Montrouge, France. Il s'agit de dispositifs à microprocesseur qui peuvent être aisément adaptés par incorporation d'éléments logiciels exécutables conçus pour mettre en oeuvre les fonctions de l'invention décrites ci-dessus. L'adaptation de ces appareils à la mise en oeuvre de l'invention étant à la portée de l'homme du métier, elle ne sera pas décrite ici en détail. Quant au signal phonocardiographique, il peut être capté et traité de manière en elle-même connue, en employant par exemple les moyens décrits dans le EP-A-0 714 627 (ELA Médical).

## Revendications

1. Un dispositif de mesure non invasive de la pression artérielle, notamment pour le suivi ambulatoire en continu de la pression artérielle,
**caractérisé en ce que** le dispositif mesure ladite pression artérielle de manière indirecte à partir des bruits émis par le coeur,
et **en ce qu'**il comprend :
- au moins un capteur (10 ; 12) apte à être placé en un point de la paroi thoracique d'un patient, et apte à transformer en un signal électrique phonocardiographique les signaux acoustiques transmis à travers le thorax engendrés par la fermeture des valves cardiaques,
- des moyens discriminateurs, aptes à reconnaître et extraire du signal phonocardiographique un profil vibratoire (18) lié au second bruit cardiaque produit périodiquement en fin de systole, et
- des moyens d'analyse, aptes à analyser au moins un paramètre prédéterminé du profil vibratoire et à délivrer en réponse, en fonction de ce(s) paramètre(s), une valeur d'indice phonoartériel représentative de la pression artérielle.

2. Le dispositif de la revendication 1, comprenant en outre des moyens d'enregistrement du signal phonocardiographique, brut ou prétraité, et dans lequel les moyens discriminateurs et d'analyse opèrent en temps différé sur le signal phonocardiographique enregistré.

3. Le dispositif de la revendication 1 ou 2, dans lequel ledit paramètre est un paramètre du groupe comprenant l'amplitude (20) séparant les extrema du signal phonocardiographique sur la durée du profil (18), l'énergie de ce signal, la variation de la dérivée de ce signal, l'aire de ce signal, ou une combinaison de ces paramètres.

4. Le dispositif de la revendication 1 ou 2, dans lequel les moyens d'analyse comportent des moyens pour appliquer au(x)dit(s) paramètre(s) une valeur de pondération variable d'un profil à l'autre.

5. Le dispositif de la revendication 4, dans lequel :
- les moyens discriminateurs sont également aptes à reconnaître et extraire du signal phonocardiographique un autre profil vibratoire (16), lié au premier bruit cardiaque produit périodiquement en début de systole,
- le dispositif comprend des moyens d'analyse d'au moins un paramètre prédéterminé de cet autre profil vibratoire, aptes à délivrer une valeur de pondération fonction de ce(s) paramètre(s).

6. Le dispositif de la revendication 1 ou 2, comprenant au moins deux capteurs, ainsi que des moyens pour combiner les signaux délivrés par ces capteurs en un signal moyen unique appliqué aux moyens discriminateurs.

7. Le dispositif de la revendication 1 ou 2, comprenant en outre des moyens d'évaluation de la position corporelle du patient, ainsi que des moyens d'apprentissage aptes à mémoriser préalablement une pluralité de niveaux moyens du signal phonocardiographique en fonction de positions corporelles correspondantes, et dans lequel les moyens d'analyse comportent des moyens pour appliquer au(x)dit(s) paramètre(s) une valeur de pondération fonction du niveau moyen mémorisé pour la position corporelle au moment de l'analyse.

8. Le dispositif de la revendication 1 ou 2, comprenant en outre des moyens de filtrage passe-bas de l'indice phonoartériel.

9. Le dispositif de la revendication 8, comprenant en outre des moyens d'évaluation de la fréquence respiratoire, et dans lequel les moyens de filtrage passe-bas sont des moyens de filtrage adaptatif à fréquence de coupure variable en fonction de la valeur de la fréquence respiratoire au moment de l'analyse.

10. Le dispositif de la revendication 1, comprenant en outre des moyens d'analyse du cycle respiratoire, ainsi que des moyens de filtrage de l'indice phonoartériel, ces moyens de filtrage étant des moyens de filtrage dynamique à gain variable en fonction de la phase du cycle respiratoire au moment de l'analyse.

11. Le dispositif de la revendication 1, comprenant en outre des autres moyens de mesure de la pression artérielle, aptes à délivrer une valeur absolue de cette pression artérielle, dans lequel ces autres moyens de mesure sont activés ou inhibés de manière sélective en fonction de la valeur d'indice phonoartériel délivrée par les moyens d'analyse.

12. Le dispositif de la revendication 1, comprenant en outre des moyens de pilotage d'un dispositif de mesure et d'analyse d'un signal électrocardiographique et/ou de mise en oeuvre d'une thérapie.

## Claims

1. A device for the non-invasive measurement of arterial pressure, in particular for the continuous ambulant monitoring of the arterial pressure, **characterised in that** the device measures said arterial pressure indirectly on the basis of noise emitted by the heart, and **in that** it comprises:
- at least one sensor (10; 12) to be placed at a point on the thoracic wall of a patient, and being able to transform the acoustic signals transmitted through the thorax and generated by the closing of the cardiac valves into an electric phonocardiographic signal,
- discriminating means for identifying and extracting from the phonocardiographic signal a vibratory profile (18) related to the second cardiac noise periodically produced at the end of the systole, and
- analysing means for analyzing at least one predetermined parameter of the vibratory profile and, in response, delivering as a function of said parameter(s), a phono-arterial index value representative of the arterial pressure.

2. The device of claim 1, further comprising means for recording the phonocardiographic signal, raw or pre-processed, and wherein the discriminating and analyzing means processes the recorded phonocardiographic signal with delay.

3. The device of claim 1 or 2, wherein said parameter is a parameter selected from among the group comprising the amplitude (20) separating the extrema of the phonocardiographic signal for the duration of the profile (18), the energy of this signal, the variation of the derivative of this signal, the surface area of this signal, or a combination of these parameters.

4. The device of claim 1 or 2, wherein the analyzing means comprises means for applying a weighted value to said parameter(s), said weighted value being variable from one profile to another.

5. The device of claim 4, wherein:
- the discriminating means is equally able to identify and extract from the phonocardiographic signal a different vibratory profile (16) related to the first cardiac noise periodically produced at the beginning of a systole,
- the device comprises means for analyzing at least one predetermined parameter of said different vibratory profile, able to deliver a weighted value that is a function of said parameter(s).

6. The device of claim 1 or 2, comprising at least two sensors, as well as means for combining the signals delivered by said sensors into a unique averaged signal applied to said discriminating means.

7. The device of claim 1 or 2, further comprising means for evaluating the body position of the patient, as well as learning means able to memorize in advance a plurality of average levels of the phonocardiographic signal as a function of corresponding body positions, and wherein the analyzing means comprises means for applying to said parameter(s) a weighted value that is a function of the average level memorized for the body position at the moment of the analysis.

8. The device of claim 1 or 2, further comprising means for low-pass filtering the phono-arterial index.

9. The device of claim 8, further comprising means for evaluating the respiratory frequency, and wherein said means for low-pass filtering is a means for adaptive filtering at a cut-off frequency that is variable as a function of the respiratory frequency value at the moment of the analysis.

10. The device of claim 1, further comprising means for analyzing the respiratory cycle, as well as means for filtering the phono-arterial index, wherein said filtering means is a means for dynamic filtering at a variable gain as a function of the phase of the respiratory cycle at the moment of the analysis.

11. The device of claim 1, further comprising different means for measuring the arterial pressure, being able to deliver an absolute value of said arterial pressure, wherein said different measuring means is activated or inhibited in a selective fashion as a function of the phono-arterial index value delivered by the analyzing means.

12. The device of claim 1, further comprising means for controlling a device for measuring and analyzing an electrocardiographic signal and/or implementation of a therapy.

## Patentansprüche

1. Vorrichtung zur nicht-invasiven Messung des Arteriendrucks, insbesondere für das ständige ambulante Verfolgen des Arteriendrucks, **dadurch gekennzeichnet, dass** die Vorrichtung den Arteriendruck auf indirekte Weise anhand der vom Herzen abgegebenen Geräusche misst, und dass sie folgendes umfasst:
- mindestens einen Sensor (10; 12), der an einem Punkt der Brustkorbwand des Patienten angeordnet werden kann, und dazu geeignet ist, die durch den Brustkorb übertragenen, durch das Schließen der Herzklappen verursachten akustischen Signale in ein elektrisches phonokardiographisches Signal umzuwandeln,
- Unterscheidungsmittel, die geeignet sind, in dem phonokardiographischen Signal ein Vibrationsprofil (18) zu erkennen und zu entnehmen, das mit dem zweiten Herzgeräusch zusammenhängt, welches regelmäßig am Ende einer Systole erzeugt wird, und
- Analysemittel, die geeignet sind, wenigstens einen vorbestimmten Parameter des Vibrationsprofils zu analysieren und in Antwort darauf in Abhängigkeit dieses Parameters (dieser Parameter) einen den Arteriendruck repräsentierenden phonoarteriellen Kennwert zu liefern.

2. Vorrichtung nach Anspruch 1, weiterhin mit Mitteln zur Speicherung des phonokardiographischen Signals, roh oder vorbearbeitet, und bei welcher die Unterscheidungs- und Analysemittel das gespeicherte phonokardiographische Signal zeitversetzt verarbeiten.

3. Vorrichtung nach Anspruch 1 oder 2, bei welcher der Parameter ein Parameter aus der Gruppe enthaltend die Amplitude (20), welche die Extrema des phonokardiographischen Signals über die Dauer des Profils (18) trennt, die Energie dieses Signals, die Veränderung der Ableitung dieses Signals, die Fläche dieses Signals, oder einer Kombination dieser Parameter ist.

4. Vorrichtung nach Anspruch 1 oder 2, bei welcher die Analysemittel Mittel umfassen, um auf den Parameter (die Parameter) einen von einem Profil zum anderen variablen Gewichtungswert anzuwenden.

5. Vorrichtung nach Anspruch 4, bei welcher:
- die Unterscheidungsmittel ebenfalls geeignet sind, in dem phonokardiographischen Signal ein anderes Vibrationsprofil (16) zu erkennen und zu entnehmen, das mit dem ersten Herzgeräusch zusammenhängt, welches regelmäßig zu Beginn einer Systolc erzeugt wird,
- die Vorrichtung Mittel zur Analyse wenigstens eines vorbestimmten Parameters dieses anderen Vibrationsprofils umfasst, die geeignet sind, einen Gewichtungswert in Abhängigkeit zu diesem Parameter (diesen Parametern) zu liefern.

6. Vorrichtung nach Anspruch 1 oder 2, mit wenigstens zwei Sensoren, sowie Mitteln, um die durch diese Sensoren gelieferten Signale zu einem einzigen durchschnittlichen Signal zu kombinieren, das an die Unterscheidungsmittel angelegt wird.

7. Vorrichtung nach Anspruch 1 oder 2, außerdem mit Mitteln zur Bestimmung der Körperstellung des Patienten, sowie Lemmitteln, die geeignet sind, vorab eine Vielzahl von mittleren Pegeln des phonokardiographisehen Signals in Abhängigkeit entsprechender Körperstellungen zu speichern, und bei welcher die Analysemittel Mittel umfassen, um auf den (die) Parameter einen Gewichtungswert abhängig vom mittleren, für die zum Zeitpunkt der Analyse bestehende Körperstellung gespeicherten Pegel anzuwenden.

8. Vorrichtung nach Anspruch 1 oder 2, weiterhin mit Mitteln zur Tiefpassfilterung des phonoarteriellen Kennwertes.

9. Vorrichtung nach Anspruch 8, weiterhin mit Mitteln zur Bestimmung der Atmungsfrequenz, und bei welcher die Tiefpassfiltermittel Mittel zur adaptiven Filterung mit variabler Grenzfrequenz in Abhängigkeit des Atmungsfrequenzwertes zum Zeitpunkt der Analyse sind.

10. Vorrichtung nach Anspruch 1, weiterhin mit Mitteln zur Analyse des Atmungszyklus, sowie Mitteln zur Filterung des phonoarteriellen Kennwerts, wobei diese Filtermittel dynamische Filtermittel mit in Abhängigkeit der zum Zeitpunkt der Analyse vorliegenden Phase des Atmungszyklus variabler Verstärkung sind.

11. Vorrichtung nach Anspruch 1, weiterhin mit anderen Mitteln zur Messung des Arteriendrucks, die geeignet sind, einen Absolutwert des Arteriendrucks zu liefern, bei welcher diese anderen Messmittel selektiv in Abhängigkeit des durch die Analysemittel gelieferten phonoarteriellen Kennwerts aktiviert oder gehemmt werden.

12. Vorrichtung nach Anspruch 1, weiterhin mit Mitteln zur Steuerung einer Vorrichtung zur Messung und zur Analyse eines elektrokardiographischen Signals und/oder zur Umsetzung einer Therapie.
